# EUROPEAN PATENT SPECIFICATION

(11) **EP 2 956 211 B1**
(45) Date of publication and mention of the grant of the patent: **18.04.2018**
(21) Application number: 14707252.4
(22) Date of filing: 18.02.2014
(51) Int. Cl.: A61N 1/32, A61N 1/36, A61F 9/00, A61N 1/04

(54) **DEVICE FOR THE TREATMENT OF AN OCULAR DISEASE**
VORRICHTUNG ZUR BEHANDLUNG EINER AUGENERKRANKUNG
DISPOSITIF POUR LE TRAITEMENT D'UNE MALADIE OCULAIRE

(30) Priority: 18.02.2013 EP 13155682
(43) Date of publication of application: 23.12.2015
(73) Proprietor: Eyevensys, 75005 Paris (FR)
(72) Inventor: LEBRETON, Luc, 21121 Daix (FR); BENARD, Romain, 75011 Paris (FR); BEHAR-COHEN, Francine, 75006 Paris (FR); TOUCHARD, Elodie, 75006 Paris (FR)
(74) Representative: Nony
(86) International application number: PCT/IB2014/059073
(87) International publication number: WO 2014/125463

(56) References cited:
- WO-A1-2013/024436
- US-A1- 2010 268 150
- US-A1- 2011 009 807
- US-A1- 2011 152 749
- US-A1- 2012 302 972

## Description

### FIELD OF THE INVENTION:

The present invention relates to an injection device for the treatment of an ocular disease in a subject.

### BACKGROUND OF THE INVENTION:

WO 2006/123248 describes a device for administering a product by electroporation.

WO 00/07530, WO 2007/052730 and WO 2006/052557 describe injection devices. US2011/152749 discloses an injection device according to the preamble of claim 1. There is a need for an efficient electroporation device which may be used to introduce a pharmaceutical composition into an eye with a very precise and stable positioning.
It is an object of the invention to provide such a device.

### SUMMARY OF THE INVENTION

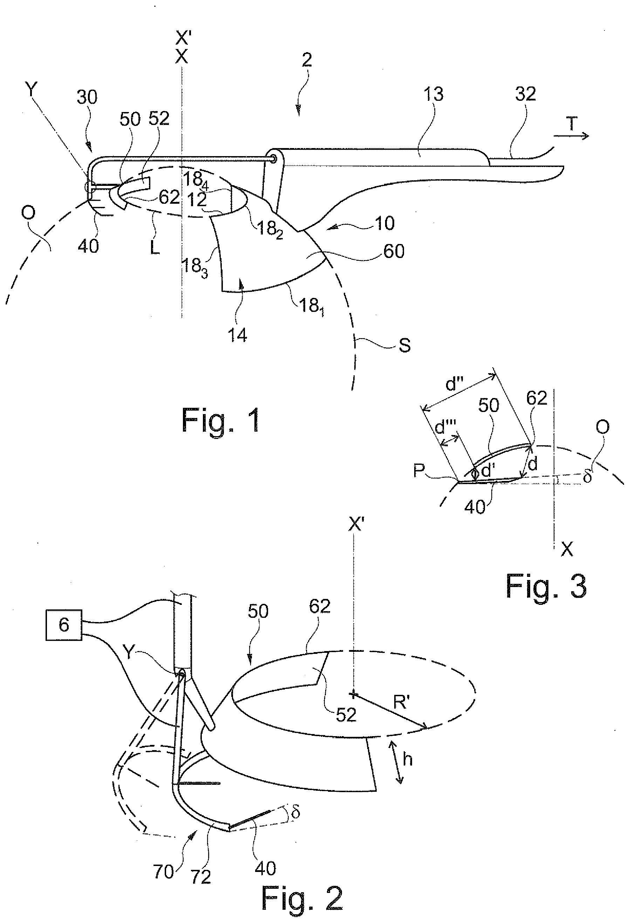

The invention is defined by the device of independent claim 1. Preferred embodiments are defined by the dependent claims. Any disclosed methods are merely exemplary. Any example or embodiment which does not fall under the scope of the claims is not part of the invention. According to a first aspect, the invention proposes an electroporation device for injecting a product into an eye, in particular the eye of a human, said device comprising:
- a first part comprising a rim having the shape of a circle or of an arc of a circle, said circle having an axis X and a radius (R') of greater than 5 mm and of less than 8 mm, so as to substantially correspond to the radius of the limbus of the eye (O), (so that it can be placed in contact with said limbus to at least partially encircle said limbus),
- a second part mobile relative to the first part and guided by said first part, said second part comprising an invasive first electrode and/or a second electrode having a spherical electrode contact surface born by a virtual sphere, the radius of curvature of said electrode contact surface ranging between 11 mm and 18 mm, being preferably between 11 mm and 14 mm, and preferably about 12.5 mm, so as to substantially correspond to the radius of curvature of the outside surface of said eye (i.e. a contact surface conformed to bear on the outside surface of said eye),
the first and second electrodes being designed to be connected electrically to first and second terminals, respectively, of an electrical generator. As will be described in more detail later in the description, the inventors have discovered that such an injection device is very efficient for electroporation in the eye, in particular in the ciliary muscle, allowing a very precise distribution of the pharmaceutical composition, in particular containing a plasmid DNA.
Preferably, an injection device according to the invention comprises one or more of the following optional characteristics:
- The first part has a spherical first part contact surface born by a virtual sphere, the radius of curvature of said spherical contact surface ranging between 11 mm and 18 mm, preferably between 11 mm and 14 mm, and being preferably about 12.5 mm, so as to substantially correspond to the radius of curvature of the outside surface of said eye (the diameter of the outside surface of a human eye is about 25 mm),
   and the area of said spherical first part contact surface is preferably greater than 30 mm², greater than 40 mm², greater than 50 mm², greater than 60 mm², greater than 80 mm², greater than 100 mm², greater than 150 mm², greater than 200 mm² and/or less than 900 mm² less than 800 mm² less than 700 mm² less than 600 mm², or less than 500 mm², and/or
   said rim preferably extends on more than 45°, preferably more than 60°, preferably more than 70°, preferably more than 80°, preferably more than 90°, preferably more than 120°, or more than 160°, more than 180°, more than 200°, more than 250°, around the axis X.
- The first and second parts are preferably configured so that they may bear on two respective areas of one circle having an axis X and a radius (R') of greater than 5 mm and of less than 8 mm, so as to substantially correspond to the radius of the limbus of the eye (O), said axis being opposite to the center of said circle.
- The second part comprises said first and second electrodes.
- The first electrode comprises one, preferably several injection needles.
- The spherical electrode contact surface is configured to come into electrical contact with the outside surface of said eye, i.e. is not electrically isolated from said outside surface of said eye.
- The second electrode comprises a rim having the shape of an arc of a circle, said circle having an axis X' and a radius of greater than 5 mm, greater than 6 mm, and/or of less than 8 mm or less than 7 mm, so as to substantially correspond to the radius of the limbus of the eye (O), (so that it can be placed in contact with said limbus to partially encircle said limbus). In the position represented in the figure 1, the axis X and X' are the same.
- The rims of the first part and of the second electrode are coplanar (or may be coplanar if the second electrode is mobile relative to the first electrode) at least in a position where the first part and the spherical surface of the second electrode bear on a same virtual sphere, the radius of curvature of said virtual sphere ranging between 11 mm and 18 mm, preferably between 11 mm and 14 mm, and being preferably about 12.5 mm, so as to substantially correspond to the radius of curvature of the outside surface of said eye. In Fig. 1, these rims bear on the same circle, corresponding to the limbus L.
- The second part is guided, relative to the first part, in rotation, in particular like two arms of a pair of scissors, and/or in translation (along the direction T, as represented), the direction T corresponding preferably to the direction of the invasive needle(s) of the first electrode.
- The first electrode is fixed relative to the second electrode or mobile relative to the second electrode.
- The first electrode is guided between a remote position and a close position in which the first electrode is close to and remote from the second electrode, respectively.
- The first electrode is guided, relative to the second electrode, in rotation and/or in translation. The rotation is preferably around an axis Y (see Fig. 1) which belongs to a plane perpendicular to the axis X and/or X', and/or which is preferably substantially tangential to a circle of axis X and/or X'. The translation is preferably along an axis which belongs to a plane perpendicular to the axis X and/or X' and/or which is preferably substantially radial to a circle of axis X and/or X'.
- The electroporation device comprises
   - elastic means, for instance a spring, configured to force the movement of second part toward the first part, and/or the movement of the first electrode relative to the second electrode, and/or
   - a mechanism to automatically change the position of the second part relative to the first part, and/or the position of the first electrode relative to the second electrode.
- A needle of the first electrode (preferably any needle of the first electrode) is protruding or or may be guided so as to protrude inside the virtual sphere (S) corresponding to the second electrode contact surface.
- Preferably, the angle δ between a needle of the first electrode (preferably any needle of the first electrode) and a plane perpendicular to the axis X or X' (see Fig. 2) is less than 20°, less than 15°, preferably less than 10°, preferably less than 5°, preferably substantially 0°, in particular when the first electrode is mobile in translation relative to the second electrode. Preferably, a needle of the first electrode (preferably any needle of the first electrode) extends, or may be guided so as to extend, substantially in a plane perpendicular to said axis X' and/or, preferably, in a plane including said axis X', i.e. said needle is substantially radial.
- The first electrode is guided between a remote position (in dashed line in Fig. 2) and a close position (in plain line in Fig. 2) or is fixed relative to the second electrode,
   and a needle of the first electrode (preferably any neddle of the first electrode) extends permanently (if the first electrode is fixed relative to the second electrode) or in the close position at a distance *d* less than 15 mm (i.e. no point of said needle is at a distance greater than 15 mm away from the rim of said second electrode), less than 10 mm, less than 8 mm, less than 7 mm, less than 6 mm, less than 5 mm, less than 4 mm, less than 3 mm, and/or greater than 0.5 mm, greater than 1 mm from said rim of said second electrode and/or at a distance d' less than 7 mm, less than 6 mm, less than 5 mm, less than 4 mm, less than 3 mm, less than 2 mm, less than 1 mm, and/or greater than 0.5 mm from said second electrode (voir Fig. 3). Preferably, the first electrode is configured so that, permanently or in the close position, no portion of said needle of the first electrode is at a distance greater than 10 mm, greater than 8 mm, greater than 7 mm, greater than 6 mm, greater than 5 mm, greater than 4 mm, greater than 3 mm, greater than 2 mm from the electrically conducticve contact surface of the second electrode.
- Permanently (if the first electrode is fixed relative to the second electrode) or in the close position, the distance *d"* between said rim of said second electrode and the point (P) of said virtual sphere inside which said needle of the first electrode protrudes or may protrude is less than 25 mm, preferably less than 20 mm, preferably less than 15 mm, preferably less than 10 mm and or more than 4 mm, more than 5 mm, and/or the distance *d'"* between said said second electrode and the point of said virtual sphere inside which said needle of the first electrode protrudes or may protrude is less than 10 mm, preferably less than 9 mm, preferably less than 8 mm, preferably less than 7 mm, preferably less than 6 mm, preferably less than 5 mm (see Fig. 3).
- A needle of the first electrode (or preferably any needle of the first electrode if it comprises several needles) is electrically insulated over a part of its length, preferably by means of an insulating cover, so that, permanently or in the close position, no non-insulated part of said needle extends at a distance less than 2.0 mm, less than 1.5 mm, less than 1.0 mm, less than 0.8 mm from the spherical contact surface of the second electrode.
- The device preferably comprises more than 1, more than 2, and /or less than 10, less than 5 or less than 4 injection needles, these injection needles being different or, preferably, identical. The injection needles are preferably part of the first electrode.
- The spherical contact surface of the second electrode and/or of the first part has the form of a spheroidal band, in particular extending along an envelope surface corresponding to the shape of the anterior or posterior part of the outside surface of an eye.
- The spherical contact surface of the second electrode and/or of the first part has a surface area of greater than 30 mm², greater than 40 mm², greater than 50 mm², greater than 60 mm², greater than 80 mm², greater than 100 mm², greater than 150 mm², greater than 200 mm², and/or less than 900 mm², less than 800 mm², less than 700 mm², less than 600 mm², or less than 500 mm².
- The first electrode comprises one or a plurality of injection needles which preferably substantially extend along a common general direction. The first electrode is preferably rotationally mounted on said second part, preferably around an axis (Y) substantially perpendicular to said general direction, the axis of rotation being preferably substantially perpendicular to the general direction of the injection needle(s).
- The device is configured so that contact surface of the second electrode may be in contact with a second region of the outside surface of the eye, preferably so as to match said second region of said outside surface, the second region being opposite to a first region (relative to the axis X) on which the contact surface of the first part bears.
- Preferably, the electrical generator is designed to promote the electroporation of a composition injected into an eye by means of injection needles of the device.

According to a second aspect, the invention provides an electroporation device, which may optionnaly have one or several characteristics of the electroporation device of the first aspect, said device comprising first and second electrodes,
wherein the first electrode is guided between a remote position and a close position or is fixed relative to the second electrode,
wherein the second electrode comprises a rim having the shape of a circle or of an arc of a circle, said circle having an axis X' and a radius of greater than 5 mm and of less than 8 mm,
wherein the second electrode has a spherical contact surface, the radius of curvature of said spherical contact surface ranging between 11 mm and 18 mm, preferably between 11 mm and 14 mm, and being preferably about 12.5 mm,
wherein the first electrode extends, or may be guided so as to extend, so that a needle of said first electrode (or preferably any needle of said first electrode) protrudes inside the virtual sphere corresponding to the second electrode contact surface ,
wherein the angle δ between said needle of the first electrode (or any needle of the first electrode) and a plane perpendicular to the axis X' is less than 20°, less than 15°, preferably less than 10°, preferably less than 5°, preferably substantially 0°.

According to a third aspect, the invention provides an electroporation device, comprising
- an invasive first electrode,
- a support having a spherical support contact surface, i.e. extending along a virtual sphere, the radius of curvature of said support contact surface ranging between 11 mm and 18 mm, preferably between 11 mm and 14 mm, and being preferably about 12.5 mm, so as to substantially correspond to the radius of curvature of the outside surface of said eye,
wherein the contact surface of said support comprises a rim having the shape of a circle (a complete circle, not an arc of a circle) having an axis X and a radius of greater than 5 mm and of less than 8 mm, so as to substantially correspond to the radius of the limbus of the eye (O) (so that it can be placed in contact and so as to encircle said limbus),
wherein the first electrode is mobile relative to the support between an extreme (i.e. limited by an abutment) close position and a remote position in which a needle of the first electrode is protruding and not protruding from the support contact surface, respectively, said the first electrode being guided during the movement between the remote position and the close position and said needle of the first electrode extending, in the close position at a distance *d1* from said rim and/or at a distance *dl'* from said support contact surface, preferably from an electrode contact surface of a second electrode born by said support.
wherein, preferably, the distance *d1* is less than 15 mm (i.e. no point of said needle of the first electrode is at a distance greater than 15 mm away from the rim), less than 10 mm, less than 8 mm, less than 7 mm, less than 6 mm, less than 5 mm, less than 4 mm, less than 3 mm, and/or greater than 0.5 mm, greater than 1 mm, and/or preferably, the distance *d1'* is less than 7 mm, less than 6 mm, less than 5 mm, less than 4 mm, less than 3 mm, less than 2 mm, less than 1 mm, and/or greater than 0.5 mm.
Preferably, the first electrode is configured so that, permanently or in the close position, no portion of said needle of the first electrode is at a distance greater than 10 mm, greater than 8 mm, greater than 7 mm, greater than 6 mm, greater than 5 mm, greater than 4 mm, greater than 3 mm, greater than 2 mm from an electrically conductive contact surface of the second electrode.
The first and second electrodes of this device may optionnaly have one or several characteristics of the first and second electrodes of the electroporation device according to the first aspect of the invention.
In a preferred embodiment, the device comprises an injection needle which is not an electrode. Preferably it comprises means to guide said injection needle into a position adapted for the injection of the composition, and in particular into a position wherein the composition is injected between the first and second electrodes.
In this embodiment, the first electrode preferably does not comprise any injection means.

### Method

The disclosure also relates to a method for injecting a composition into an eye by means of an electroporation device according to the first aspect of the invention, said method comprising the following steps:
a) positioning the first part on the eye so that the rim of said first part bear on the limbus of the eye, preferably so that the spherical contact surface of said first part, if any, comes into contact with a first region of the outside surface of the eye,
b) positioning the second part so that the invasive first electrode penetrates into the eye and/or the spherical electrode contact surface of the second part comes into contact with a second region of the outside surface of the eye, the second region being preferably, relative to the axis X, substantially opposite to the rim of said first part and, if any, opposite to the first region,
c) if the first electrode and the second electrode are mobile relative to each other, moving the first electrode or the second electrode so as to reach a position where the invasive first electrode is inserted in the eye and the spherical electrode contact surface of the second part is in contact with a second region of the outside surface of the eye,
d) independently of the preceding steps, injecting said composition through at least an injection needle, preferably through the first electrode,
e) independently of the preceding steps, connecting the first and second electrodes to said first and second terminals, respectively,
f) generating an electrical field between said first and second electrodes with said electrical generator, the electrical field being adapted to promote electroporation.

### BRIEF DESCRIPTION OF THE FIGURES

Other features and advantages of the invention will become clear upon reading the following detailed description and by examining the attached drawing, in which:
- Figure 1 shows an electroporation device according to the invention;
- Figure 2 shows a perspective view of a detail of an injection device of Fig. 1;
- Figure 3 shows, along a transverse plane containing the axis X and a needle of the first electrode, a partial cross section of the device shown in Figures 1 and 2.

In the various figures, identical reference signs are used to designate identical or similar elements.

### DEFINITIONS

A "spherical contact surface" means a substantially spherical contact surface, preferably so as to correspond to the shape of the anterior or posterior part of the outside surface of an eye.

"First" and "second" are used to distinguish corresponding elements, but do not limitate the invention.

In the present description, unless otherwise stated, "comprising a" should be understood as "comprising at least one".

### DETAILED DESCRIPTION

The electroporation device 2 shown in Figure 1 is configured to catch in a pincer movement an eye O, while bearing on the limbus L.

The device comprises a first part 10 and a second part 30, mounted in translation on the first part 10, along a direction T.

### First part

The first part 10 comprises a proximal portion 13 intended for manipulation of the device, allowing the device to be gripped, and a cup-shaped support 14 mounted at the end of the proximal portion 13.

The support 14 is provided with a spherical first part contact surface 60 intended to come into contact with the outside surface of the eye, to ensure an efficient and stable positioning of the device.

The contact surface 60 preferably has a smooth surface and, more preferably, a surface without any roughness, preferably in a material that is not aggressive with respect to the surface of the eye, for example polymers of silicone, of sponge, in particular synthetic sponge, of polyester, of polyorthoester, of polymethyl methacrylate or of any other flexible medical-grade polymers.

The support 14 is preferably rigid.

In the represented embodiment, the contact surface 60 extends along the substantially spherical envelope S, matching the outside surface of an eye O.The contact surface 60 may have the form of a band.

The contact surface 60 may have two large sides 18₁ and 18₂ and two small sides 18₃ and 18₄. The large sides can in particular form rounded corners with the small sides.

The length of the small sides and/of the large sides may be greater than or equal to 5 mm, greater than or equal to 6 mm, and/or less than 20 mm, less than 18 mm, less than 15 mm, or less than 12 mm.

The contact surface 60 may be solid or may be locally perforated (e.g. by holes). Preferably, the contact surface 60 is continuous, i.e. is not perforated.

The contact surface 60 has a rim 12 designed to bear along the limbus L, i.e. the transition shoulder between the cornea and the sclera. The rim 12 allows the operator to position the first part, with remarkable precision, on the surface of the eye before any penetration of the injection needles through the outside surface thereof. The risk of error is therefore reduced or substantially eliminated.

The rim 12 may be continuous or not.

The arc of a circle of the rim 12 may have a radius R' of greater than 5 mm, greater than 6 mm, and/or of less than 8 mm or less than 7 mm, a radius of 6.58 mm being prefered.

The arc of a circle of the rim 12 may extend, around its axis, on more than 40°, more than 50°, more than 60°, more than 70°, more than 80°, more than 90°, more than 100°, more than 120°, more than 140°, more than 160°. In an embodiment, it may extend on more than 180°, more than 200°, more than 220°, more than 240°, more than 260°, more than 280°, more than 300°, or even more, i.e. up to 360°.

The rim 12 preferably has a smooth surface and, more preferably, a surface without any roughness, especially in the form of sharp tips or edges that could damage the surface of the eye.

The rim 12 may in particular be formed by a band of flexible material with a width of greater than 1.5 mm and/or less than 5 mm. It may in particular be formed by a bead of silicone or of foam. Advantageously, the risk of injury to the limbus is thereby reduced.

The rim 12 may be formed by or at least partially covered by a non-slip material that is able to limit the sliding movement on the outside surface of the eye.

### Second part

The second part 30 comprises an invasive first electrode 40 and a second electrode 50 having a spherical electrode contact surface 52. The contact surface 52 is defined by an electrically conductive material and may have one or several of the features of the contact surface 60. In particular, the contact surface 52 may be defined by a coating made of an electrically conductive material.

The height *h* of the electrode contact surface 52, i.e. the length of its small edges, may be less than 10 mm, less than 8 mm, less than 7 mm, less than 5 mm.

The electrode contact surface 52 may have a rim 62, intended to come into contact with the limbus L, so as to perfectly position and stabilize the device on the eye. The rim 62 may have one or several of the features of the rim 12.

The electrode contact surface 52 preferably does not comprise any injection needle, i.e. the electrode 52 is preferably a surface electrode, i.e. designed so as to not penetrate into the eye.

The second part comprises a first connector for connecting the first electrode to a first terminal of an electrical generator 6. The second part 30 also comprises a second connector for connecting the electrode contact surface 52, defined by an electrically conductive material, to a second terminal of the electrical generator 6.

The device is preferably configured so that the contact surfaces 60 and 52 are concentric in a position where they bear on opposite regions of the outside surface of the eye, relative to the vision axis of the eye, as shown in Figure 1. The radius of curvature of the contact surface(s) 60 and/or 52 ranges between 11 mm and 18 mm. It may range between 15 mm and 18 mm, but, for a human eye, preferably ranges between 11 mm and 18 mm and preferably ranges between 11 mm and 14 mm.

The second part 30 may comprise a second proximal portion 32, facilitating the manipulation of the second part, in particular to guide the movement along the direction T. The second electrode 50 may be rigidly fixed at the end of the second part which is opposite to the proximal portion 32.

The translation movement of the second part relative to the first part along the direction T may lead the second part in a position where the first and second contact surfaces 52 and 60 may "pinch" or "clamp" the eye O, two opposite regions of the outside surface of the eye O (relatively to the axis X) being in close contact with the contact surfaces 52 and 60, respectively.
A spring preferably tends to push the first part toward the second part.
The second part also preferably comprises a first invasive electrode 70, comprising at least one needle 40, preferably a set of parallel identical and rectilinear needles 4', extending along a common general direction T, and electrically connected to each other. The needles 40 are fixed on a common stem 72, as a comb. The needle(s) 40 is (are) preferably injection needles.
The external diameter of a needle 40 may be between 0.2 and 0.4 mm.
The external diameter of a needle 40 is, for example, about 0.3 mm.
The distal end of a needle has preferably a bevelled tip for facilitating the penetration of the needle into the eye. It may open out *via* one or several axial and/or radial ejection orifices. In an embodiment, any needle 40 is tapered, that is to say conical along its axis, and opens out axially.
Preferably, the ejection orifice of an injection needle, preferably of any injection needle, has a smaller diameter than the inside diameter of the injection needle. Preferably, the injection needle opens out laterally.
A needle 40 can be designed in such a way that its distal end reach the ciliary muscle or the vitreous cavity, or the subretinal space, or the retina, or the pigment epithelium of the retina, or the corneal epithelium or stroma or endothelium or transcornea, or else the peri-ocular muscles. Means are preferably provided to ensure that this distal end cannot be engaged beyond these elements during the stage of penetration.
The length of a needle 40 can be greater than 2 mm, or greater than 3 mm, or greater than 3.3 mm and/or less than 5 mm, or less than 4 mm, or less than 3.7 mm. All the needles may have the same dimensions.
Preferably, the needles are configured so that, in the close position (Fig. 1), with the electrode contact surface bearing on the eye and the rim 62 on the limbus, no needle may penetrate into the eye at a distance less than 4 mm, preferably less than 5 mm from the limbus. In particular, all the fixation points of the needles on the stem 72 are preferably more than 4 mm, preferably more than 5 mm away from the rim 62.

The first electrode is mounted on the second part so that it may rotate or translate between a remote position where the needles 40 are not penetrating in the eye (represented with a dashed line) and a close position where the needles 40 are introduced in the eye, while allowing the rim 62 remaining in contact with the limbus of the eye (and the spherical surface 52 remaining in contact with the outside surface of the eye) during the rotation or translation between these two positions.

The device preferably comprises desactivable means to lock the first electrode in the remote position and/or in the close position. The device may comprise elastic means, for instance a spring, acting so as to push the first electrode toward the close position and/or to push the second electrode onto the outside surface of the eye and/or to push the second part toward the first part. Such elastic means advantageously improve the stability of the positioning.

The electroporation device preferably comprises a reservoir of the pharmaceutical composition that is to be injected, for instance a reservoir in the form of a syringe, in fluid communication with all the injection needle(s). An action on the piston of the syringe transfers the pharmaceutical composition out of the reservoir into the injection needle(s).

### Pharmaceutical composition

An electroporation device according to the invention may be used for the electroporation of a therapeutic nucleic acid of interest.

The nucleic acid to be used in the instant invention can be any nucleic acid of interest exhibiting a biological property. More particularly, the nucleic acid can be any nucleic acid encoding a natural, truncated, artificial, chimeric or recombinant product [e.g., a polypeptide of interest (including a protein or a peptide), a RNA, etc.] exhibiting a biological activity.

The nucleic acid is preferably a desoxyribonucleic acid (DNA) molecule (cDNA, gDNA, synthetic DNA, artificial DNA, recombinant DNA, etc.) or a ribonucleic acid (RNA) molecule (mRNA, tRNA, RNAi, RNAsi, catalytic RNA, antisens RNA, viral RNA, etc.). The nucleic acid may be single stranded or multistranded nucleic acid, preferably double-stranded nucleic acid or may be complexed. The nucleic acid may comprise hybrid sequences or synthetic or semi-synthetic sequences. It may be obtained by any technique known to persons skilled in the art, and especially by screening libraries, by chemical synthesis, or alternatively by mixed methods including chemical or enzymatic modification of sequences obtained by screening libraries.

In a particular embodiment, the therapeutic nucleic acid is of synthetic or biosynthetic origin, or extracted from a virus or from a unicellular or pericellular eukaryotic or prokaryotic organism.

The therapeutic nucleic acid used in the present invention may be naked, may be complexed with any chemical, biochemical or biological agent, may be inserted in a vector, etc..

As used herein, the term "naked DNA" refers to any nucleic acid molecule which is not combined with a synthetic, biosynthetic, chemical, biochemical or biological agent improving the delivery or transfer of said DNA, or facilitating its entry into the cell.

As used herein, the term "vector" refers to a nucleic acid molecule capable of transporting another nucleic acid to which it has been linked. This term also refers in the present application to any delivery carrier, such as a composition associated to a therapeutic or prophylactic nucleic acid in order to increase its cellular delivery.

Preferred vectors are those capable of autonomous replication and/or expression of nucleic acids to which they are linked. Vectors capable of directing the expression of genes to which they are operatively linked are referred to herein as "expression vectors". In general, expression vectors of utility in recombinant DNA techniques are often in the form of "plasmids" which refer to circular double stranded DNA loops which, in their vector form, are not bound to the chromosome. In the present invention, the plasmid is the most commonly used form of vector. The plasmid is a preferred form of naked DNA according to the invention.

Vectors may also be episomal DNA, yeast artificial chromosomes, minichromosomes or viral vectors wherein the viral vector is selected from the group consisting of a lentivirus, an adenovirus, an adeno-associated virus and a virus-like vector.

The vector may also be a lipid vesicle such as a liposome. Lipid based compounds which are not liposomes may further be used. For example, lipofectins and cytofectins are lipid-based positive ions that bind to negatively charged nucleic acid and form a complex that can ferry the DNA across a cell membrane. The invention is intended to include such other forms of expression vectors which serve equivalent functions and which become known in the art subsequently hereto.

In addition, the nucleic acid according to the invention may also contain one or more additional regions, for example regulatory elements of small or large size which are available to the skilled artisan such as a promoter region (constitutive, regulated, inducible, tissue-specific, etc.), for example sequences allowing and/or promoting expression in the targeted tissue (e.g. choroid or retina) or cells (e.g. RPE or photoreceptors), a transcription termination signal, secretion sequences, an origin of replication and/or nuclear localization signal (nls) sequences which further enhance polynucleotide transfer to the cell nucleus. Such nls sequences have been described in the prior art including the SV40 large T antigen sequence.

Additionally, the nucleic acid may further comprise selectable markers useful in selecting, measuring, and monitoring nucleic acid transfer results (transfer to which tissues, duration of expression, etc.). The types of expression systems and reporter genes that can be used or adapted for use are well known in the art. For example, genes coding for a luciferase activity, an alkaline phosphatase activity, or a green fluorescent protein activity are commonly used.

The nucleic acid according to the invention may contain any nucleotide sequence of any size. The nucleic acid may thus vary in size from a simple oligonucleotide to a larger molecule such as a nucleotide sequence including exons and/or introns and/or regulatory elements of any sizes (small or large), a gene of any size, for example of large size, or a chromosome for instance, and may be a plasmid, an episome, a viral genome, a phage, a yeast artificial chromosome, a minichromosome, an antisense molecule, etc.

In a particularly preferred embodiment, the polynucleotide is a double-stranded, circular DNA, such as a plasmid, encoding a product with biological activity.

The nucleic acid can be prepared and produced according to conventional recombinant DNA techniques, such as amplification, culture in prokaryotic or eukaryotic host cells, purification, etc. The techniques of recombinant DNA technology are known to those of ordinary skill in the art.

In a particular embodiment, the nucleic acid of interest is capable of exerting a beneficial effect on the targeted cells. It may compensate for a deficiency in or reduce an excess of an endogenous substance. Alternatively, it may confer new properties on the targeted cells. It may be for example an antisense sequence or nucleic acid encoding a polypeptide which can affect the function, morphology, activity and/or metabolism of ocular cells.
The down regulation of gene expression using antisense nucleic acids can be achieved at the translational or transcriptional level. Antisense nucleic acids of the invention are preferably nucleic acid fragments capable of specifically hybridizing with a nucleic acid encoding an endogenous ocular active substance or the corresponding messenger RNA. These antisense nucleic acids can be synthetic oligonucleotides, optionally modified to improve their stability and selectivity. They can also be DNA sequences whose expression in the cell produces RNA complementary to all or part of the mRNA encoding an endogenous ocular active substance. Antisense nucleic acids can be prepared by expression of all or part of a nucleic acid encoding an endogenous ocular active substance, in the opposite orientation. Any length of antisense sequence is suitable for practice of the invention so long as it is capable of down-regulating or blocking expression of the endogenous ocular active substance. Preferably, the antisense sequence is at least 20 nucleotides in length. The preparation and use of antisense nucleic acids, DNA encoding antisense RNAs and the use of oligo and genetic antisense is disclosed in WO92/15680. Among the biologically active polypeptides or proteins optionally expressed by a nucleic acid as described above and suitable for practice of the invention are enzymes, blood derivatives, hormones, lymphokines, cytokines, chimiokines, antiinflammatory factors, growth factors, trophic factors, neurotrophic factors, haematopoietic factors, angiogenic factors, anti-angiogenic factors, inhibitors of metalloproteinase, regulators of apoptosis, coagulation factors, receptors thereof, in particular soluble receptors, a peptide which is an agonist or antagonist of a receptor or of an adhesion protein, antigens, antibodies, fragments or derivatives thereof and other essential constituents of the cell, proteins involved in the visual cycle within RPE cells, and structure proteins of retinal cells.

Various retina-derived neurotrophic factors have the potential to rescue degenerating photoreceptor cells, and may be delivered trough a method according to the present invention. Preferred biologically active agents may be selected from VEGF, Angiogenin, Angiopoietin-1, DeM, acidic or basic Fibroblast Growth Factors (aFGF and bFGF), FGF-2, Follistatin, Granulocyte Colony-Stimulating factor (G-CSF), Hepatocyte Growth Factor (HGF), Scatter Factor (SF), Leptin, Midkine, Placental Growth Factor (PGF), Platelet-Derived Endothelial Cell Growth Factor (PD- ECGF), Platelet-Derived Growth Factor-BB (PDGF-BB), Pleiotrophin (PTN), RdCVF (Rod-derived Cone Viability Factor), Progranulin, Proliferin, Transforming Growth Factor-alpha (TGF-alpha), Transforming Growth Factor-beta (TGF-beta), Tumor Necrosis Factor-alpha (TNF-alpha), Vascular Endothelial Growth Factor (VEGF), Vascular Permeability Factor (VPF), CNTF, BDNF, GDNF, PEDF, NT3, BFGF, angiopoietin, ephrin, EPO, NGF, IGF, GMF, aFGF, NT5, Gax, a growth hormone, [alpha]-1 -antitrypsin, calcitonin, leptin, an apolipoprotein, an enzyme for the biosynthesis of vitamins, hormones or neuromediators, chemokines, cytokines such as IL-1, IL-8, IL-10, IL-12, IL-13, a receptor thereof, an antibody blocking any one of said receptors, TIMP such as TIMP-1, TIMP-2, TIMP-3, TIMP-4, angioarrestin, endostatin such as endostatin XVIII and endostatin XV, ATF, angiostatin, a fusion protein of endostatin and angiostatin, the C- terminal hemopexin domain of matrix metalloproteinase-2, the kringle 5 domain of human plasminogen, a fusion protein of endostatin and the kringle 5 domain of human plasminogen, the placental ribonuclease inhibitor, the plasminogen activator inhibitor, the Platelet Factor-4 (PF4), a prolactin fragment, the Proliferin-Related Protein (PRP), the antiangiogenic antithrombin III, the Cartilage-Derived Inhibitor (CDI), a CD59 complement fragment, vasculostatin, vasostatin (calreticulin fragment), thrombospondin, fibronectin, in particular fibronectin fragment gro-beta, an heparinase, human chorionic gonadotropin (hCG), interferon alpha/beta/gamma, interferon inducible protein (IP-10), the monokine-induced by interferon-gamma (Mig), the interferon-alpha inducible protein 10 (IP10), a fusion protein of Mig and IP10, soluble Fms-Like Tyrosine kinase 1 (FLT-1) receptor, Kinase insert Domain Receptor (KDR), regulators of apoptosis such as Bcl-2, Bad, Bak, Bax, Bik, BcI-X short isoform and Gax, fragments or derivatives thereof and the like.

In a particular embodiment, the nucleic acid encodes a soluble fragment of the TNF[alpha] receptor, the TGF[beta]2 receptor, of VEGFR-1, VEGFR-2, VEGFR-3, CCR2 or MIP1. The nucleic acid may also, in another preferred embodiment, encode an antibody, a variable fragment of a single-chain antibody (ScFv) or any other antibody fragment having recognition capacities for the purposes of immunotherapy.

In a particular embodiment of the present invention, the biologically active nucleic acid encodes a precursor of a therapeutic protein usable in the present invention such as those described above.

In another particular embodiment, the electroporation device of the invention is particularly suitable for performing gene replacement. Accordingly the nucleic acid may encode for a viable protein so as to replace the defective protein which is naturally expressed in the targeted tissue. Typically, defective genes that may be replaced include, but are not limited to, genes that are responsible for retinal degenerative diseases such as retinitis pigmentosa (RP), Leber congenital amaurosis (LCA), recessive RP, Dominant retinitis pigmentosa, X-linked retinitis pigmentosa, Incomplete X-linked retinitis pigmentosa, dominant, Dominant Leber congenital amaurosis, Recessive ataxia, posterior column with retinitis pigmentosa, Recessive retinitis pigmentosa with para-arteriolar preservation of the RPE, Retinitis pigmentosa RP12, Usher syndrome, Dominant retinitis pigmentosa with sensorineural deafness, Recessive retinitis punctata albescens, Recessive Alström syndrome, Recessive Bardet-Biedl syndrome, Dominant spinocerebellar ataxia w/ macular dystrophy or retinal degeneration, Recessive abetalipoproteinemia, Recessive retinitis pigmentosa with macular degeneration, Recessive Refsum disease, adult form, Recessive Refsum disease, infantile form, Recessive enhanced S-cone syndrome, Retinitis pigmentosa with mental retardation, Retinitis pigmentosa with myopathy, Recessive Newfoundland rod-cone dystrophy, Retinitis pigmentosa sinpigmento, Sector retinitis pigmentosa, Regional retinitis pigmentosa, Senior-Loken syndrome, Joubert syndrome, Stargardt disease, juvenile, Stargardt disease, late onset, Dominant macular dystrophy, Stargardt type, Dominant Stargardt-like macular dystrophy, Recessive macular dystrophy, Recessive fundus flavimaculatus, Recessive cone-rod dystrophy, X-linked progressive cone-rod dystrophy, Dominant cone-rod dystrophy, Cone-rod dystrophy; de Grouchy syndrome, Dominant cone dystrophy, X-linked cone dystrophy, Recessive cone dystrophy, Recessive cone dystrophy with supernormal rod electroretinogram, X-linked atrophic macular dystrophy, X-linked retinoschisis, Dominant macular dystrophy, Dominant radial, macular drusen, Dominant macular dystrophy, bull's-eye, Dominant macular dystrophy, butterfly-shaped, Dominant adult vitelliform macular dystrophy, Dominant macular dystrophy, North Carolina type, Dominant retinal-cone dystrophy 1, Dominant macular dystrophy, cystoid, Dominant macular dystrophy, atypical vitelliform, Foveomacular atrophy, Dominant macular dystrophy, Best type, Dominant macular dystrophy, North Carolina-like with progressive, Recessive macular dystrophy, juvenile with hypotrichosis, Recessive foveal hypoplasia and anterior segment dysgenesis, Recessive delayed cone adaptation, Macular dystrophy in blue cone monochromacy, Macular pattern dystrophy with type II diabetes and deafness, Flecked Retina of Kandori, Pattern Dystrophy, Dominant Stickler syndrome, Dominant Marshall syndrome, Dominant vitreoretinal degeneration, Dominant familial exudative vitreoretinopathy, Dominant vitreoretinochoroidopathy; Dominant neovascular inflammatory vitreoretinopathy, Goldmann-Favre syndrome, Recessive achromatopsia, Dominant tritanopia, Recessive rod monochromacy, Congenital red-green deficiency, Deuteranopia, Protanopia, Deuteranomaly, Protanomaly, Recessive Oguchi disease, Dominant macular dystrophy, late onset, Recessive gyrate atrophy, Dominant atrophia greata, Dominant central areolar choroidal dystrophy, X-linked choroideremia, Choroidal atrophy, Central areolar, Central, Peripapillary, Dominant progressive bifocal chorioretinal atrophy, Progresive bifocal Choroioretinal atrophy, Dominant Doyne honeycomb retinal degeneration (Malattia Leventinese), Amelogenesis imperfecta, Recessive Bietti crystalline corneoretinal dystrophy, Dominant hereditary vascular retinopathy with Raynaud phenomenon and migraine, Dominant Wagner disease and erosive vitreoretinopathy, Recessive microphthalmos and retinal disease syndrome; Recessive nanophthalmos, Recessive retardation, spasticity and retinal degeneration, Recessive Bothnia dystrophy, Recessive pseudoxanthoma elasticum, Dominant pseudoxanthoma elasticum; Recessive Batten disease (ceroid-lipofuscinosis), juvenile, Dominant Alagille syndrome, McKusick-Kaufman syndrome, hypoprebetalipoproteinemia, acanthocytosis, palladial degeneration; Recessive Hallervorden-Spatz syndrome; Dominant Sorsby's fundus dystrophy, Oregon eye disease, Kearns-Sayre syndrome, Retinitis pigmentosa with developmental and neurological abnormalities, Basseb Korenzweig Syndrome, Hurler disease, Sanfilippo disease, Scieie disease, Melanoma associated retinopathy, Sheen retinal dystrophy, Duchenne macular dystrophy, Becker macular dystrophy, and Birdshot Retinochoroidopathy. Examples of genes include but are not limited to genes encoding for ATP-binding cassette transporter, RPE65, RdCVF, CP290...

In another embodiment, the electroporation device of the invention is particularly suitable for performing exon skipping for restoring the function of mutated proteins responsible for retinal degenerative disease. Exon skipping involves blocking or preventing the incorporation into mature mRNA of one or more targeted exon(s) which encodes amino sequences that are responsible for a protein dysfunction. This is accomplished by exposing the pre-mRNA that includes exons encoding the protein to antisense oligonucleotides (AONs) which are complementary to sequence motifs that are required for correct splicing of the one or more targeted exons. The AONs bind to complementary required sequences in the pre-mRNA and prevent normal splicing. Instead, the targeted exons are excised and are not included in the mature mRNA that is translated into protein, and the amino acid sequences encoded by the targeted exons are missing from the translated protein.

Furthermore, in another embodiment of the present invention, a mixture of nucleic acids encoding distinct biologically active products can be used. This variant allows co-expression of different products in the ocular cells.

The pharmaceutical composition of the invention may also comprise compatible or physiologically acceptable carrier, excipient or diluent.

The term "pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a nontoxic solid, semi-solid or liquid filler, diluent, encapsulating material or formulation auxiliary of any type.

Pharmaceutically compatible or physiologically acceptable carrier, excipient or diluent includes diluents and fillers which are pharmaceutically acceptable for the methods of the invention, are sterile, and may be selected from neutral to slightly acidic, isotonic, buffered saline (including phosphates, chloride, etc.), aqueous or oleaginous solutions or suspensions and more preferably from sucrose, trehalose, surfactants, proteins and amino acids. The pharmaceutically compatible or physiologically acceptable carrier, excipient or diluent is preferably formulated using suitable dispersing, wetting, suspending, soothing, isotonic or viscosity building agents, stabilizers, preservatives and appropriate buffers to form an isotonic solution. The particular pharmaceutically acceptable carrier and the ratio of active compound to carrier are determined by the solubility and chemical properties of the composition, the particular mode of administration, and standard pharmaceutical practice. Those skilled in the art will understand how to formulate such vehicles by known techniques.

An example of stabilizers is disodium edetate or the like. Examples of isotonic agents are glycerin, propylene glycol, polyethylene glycol, sodium chloride, potassium chloride, sorbitol and mannitol or the like. Examples of buffers are citric acid, sodium hydrogenphosphate, glacial acetic acid and trometamol or the like. Examples of pH adjusters are hydrochloric acid, citric acid, phosphoric acid, acetic acid, sodium hydroxide, sodium carbonate and sodium hydrogencarbonate or the like. An example of soothing agents is benzyl alcohol or the like. Examples of preservatives are benzalkonium chloride, benzethonium chloride, p-hydroxybenzoate esters, sodium benzoate and chlorobutanol or the like.

Viscosity greater than that of simple aqueous solutions may be desirable to increase ocular absorption of the active compound, to decrease variability in dispensing the formulations, to decrease physical separation of components of a suspension or emulsion of formulation and/or otherwise to improve the ophthalmic formulation. Such viscosity building agents include, for example, polyvinyl alcohol, polyvinyl pyrrolidone, methyl cellulose, hydroxypropyl methylcellulose, hydroxyethyl cellulose, carboxymethyl cellulose, hydroxypropyl cellulose or other agents known to those skilled in the art. Such agents are typically employed at a level of from about 0.01 to about 2 wt. %.

Preparation forms of the pharmaceutical composition may be liquid preparations. The liquid preparations can be prepared, for example, by dissolving the biologically active agent in BSS (Balanced Salt Solution), a glycerin solution, a hyaluronic acid solution and the like. A particular composition comprises for example BBS (60%) and hyaluronic acid (40%). A stabilizer, an isotonic agent, a buffer, a pH adjustor, a soothing agent, a preservative, an injectable viscuous polymer, such as a polyorthoester or a polyanhydride, electrolytes, such as sodium, potassium, calcium, magnesium and/or chloride or the like may optionally be added in an adequate amount to the liquid preparations.

The pharmaceutical composition may comprise or the biologically active agent may be combined (in a use according to the present invention) with any additional active ingredient or adjuvant. The adjuvant may be selected from any substance, mixture, solute or composition facilitating or increasing the biological activity of the prophylactic or therapeutic agent such as any biologic, synthetic or biosynthetic agent which improves the delivery or transfer of said agent and may be assimilated to a vector (as delivery carrier) according to the invention. The adjuvant may be conditioned and administered separately or sequentially from the prophylactic or therapeutic agent containing composition and/or at a distinct site of injection. Treatment with multiple agents and/or adjuvants according to the invention need not be done using a mixture of agents and/or adjuvants but may be done using separate pharmaceutical preparations. The preparations need not be delivered at the same exact time, but may be coordinated to be delivered to a patient during the same period of treatment, i. e., within a week or a month of each other.

Any suitable therapeutic agents can be coordinated with the compositions of the present invention. Non-limiting examples of therapeutic agents which may be administered in addition to the above biologically active (prophylactic or therapeutic) agent(s) through a method according to the present invention also include permeabilizing agents such as a virus, a lipid vesicle, hyaluronic acid, lipid-based positive ions, polycationic emulsions, cationic peptides, polyplex, etc.; Actual dosage levels of active ingredients in the compositions of the present invention may be adapted so as to obtain an amount of active ingredient that is effective to obtain a desired biological activity. It should be understood, however, that the specific dose level for any particular patient will depend upon a variety of factors including the body weight, general health, sex, diet, time, rates of absorption and excretion, combination with other drugs and the severity of the particular disease being treated.

### Kit

In accordance with the present invention, kits are envisioned. A device according to the invention and a pharmaceutical composition according to the invention, and optionally instructions for use may be supplied together in a kit. Within the kit, the components may be separately packaged or contained.
Instructions can be in written, video, or audio form, and can be contained on paper, an electronic medium, or even as a reference to another source, such as a website or reference manual.
Other components such as excipients, carriers, other drugs or adjuvants, instructions for administration of the active substance or composition, and administration or injection devices can be supplied in the kit as well.

### Method

The disclosed method may be used for treating an ocular disease in a subject, the pharmaceutical composition being preferably chosen among the pharmaceutical compositions which are described here above.

### Operation

To use the electroporation device according to the first aspect of the invention, an operator may proceed by the following steps:
First, the operator couples a syringe filled with the pharmaceutical product, and electrically connects the first and second connectors to the two terminals of the electrical generator 6.
To position the injection device on the eye O, the operator pulls the first and second parts apart so that the spherical first part contact surface 60 and the spherical electrode contact surface 52 be spaced from each other. He also pull the first electrode apart from the spherical electrode surface 52, reaching the "remote" position. Preferably, in the remote position, the first electrode and the spherical electrode surface 52 are spaced from each other by a distance of at least 30 mm, preferably at least 40 mm, and/or less than 80 mm, or less than 60 mm.

The operator may then position the rim 12 on the limbus L, then position the spherical first part contact surface 60 onto the eye. Then he may push the second electrode toward the eye, so that the rim 62 be in contact on the limbus L and the spherical electrode contact surface 52 bear on the eye. The device is then perfectly and precisely stabilized on the eye, the needles 40 not penetrating in the eye, and being preferably locked in this remote position. Spring means may improve this stability.

The operator then unlocks (if necessary) and rotates the first electrode around the axis Y, to cause the needles 40 to penetrate through the outside surface of the eye. The needles penetrate substantially simultaneously through this surface, until the common stem 72 comes into contact with the outside surface of the eye.

The device is then in the "close" position, the eye O being pressed between the spherical contact surfaces 52 and 60, as shown in Figure 1, the needles being inserted precisely into the eye.

The operator then knows that the pharmaceutical product will be properly injected into the eye.

The operator then immobilizes the injection device in this position. The bearing of the first and second contact surfaces 52 and 60 on the sclera and the insertion of the needles in the eye O provide good stability of the device.

The operator may then begin the injection of the pharmaceutical product by acting on the piston of the syringe.

The increase in the local pressure in the area of the injection points is believed to promote the introduction of the injected product into the cells, particularly in the case of transfection. A person skilled in the art therefore generally considers it preferable to limit the number of injection points, if possible by using only a single injection needle. Surprisingly, however, the inventors have found that multiplication of the injection points promotes the penetration of the product.

The operator then sends a suitable electrical signal by means of the electrical generator, in such a way as to create, within the injection zone, an electrical field that promotes electroporation. The arrangement and the shapes of the first and second electrodes make it possible to create an electrical field particularly effective for electroporation.

In a particular embodiment, an electrical field constituted by one or more electrical pulse(s) is applied.

The field intensity of which is preferably between about 1 and 600 Volts, preferably 1 and 400 Volts, even more preferably between about 1 and 200 Volts, advantageously between about 10 and 100 Volts, or 15 and 70 Volts.

The total duration of application of the electric field may be between 0.01 millisecond and 1 second, preferably between 0.01 and 500 milliseconds, more preferably between 1 and 500 milliseconds, even more preferably greater than 1 or 10 milliseconds. In a preferred embodiment, the total duration of application of the electric field is between 10 milliseconds and 100 milliseconds and is preferably of 20 milliseconds.

The number of electric pulses applied may be between for example 1 and 100 000. Their frequency may be comprised between 0.1 and 1000 hertz. It is preferably a regular frequency.

Electric pulses may also be delivered in an irregular manner relative to each other, the function describing the intensity of the electric field as a function of the time for one pulse being preferably variable.

Electric pulses may be unipolar or bipolar wave pulses. They may be selected for example from square wave pulses, exponentially decreasing wave pulses, oscillating unipolar wave pulses of limited duration, oscillating bipolar wave pulses of limited duration, or other wave forms. Preferentially, electric pulses comprise square wave pulses or oscillating bipolar wave pulses.

When the electroporation of the pharmaceutical product has been completed, the operator electrically disconnects the electrodes and the generator and then moves the first electrode into the remote position (dashed line), so as to withdraw the injection needles from the eye. He then moves the first and second parts away from each other and removes the device.

As will now be clear, the device according to the invention permits
- precise and stable positioning of the electrodes and of the injection needle(s);
- precise guidance of the invasive electrode during its penetration into the eye;
- precise injection into the eye relative to the limbus;
- high stability of the first and second electrodes during the electroporation, in a position enabling the generation of an efficient large electrical field, exactly where the pharmaceutical product has been injected.

Of course, the invention is not limited to the embodiments described and shown, which have been provided by way of illustration.

In particular, the shape of an needle is not limited. The needle may be substantially rectilinear. It may also extend along an arc of a circle, in particular in order to facilitate its insertion by rotation of the first electrode around the axis Y.

The needles may be parallel or not. But it is preferable that they are substantially parallel.

The needles may have an identical length, whichever needle is considered. They may also have different length.

Moreover, the movement of the first electrode relative to the second electrode and/or the movement of the first part relative to the second part may be guided differently.

In an embodiment, the first electrode extends, or may be guided so as to extend, along a direction which is substantially parallel to the contact surface of the second electrode.

In an embodiment, the first and second parts are fixed relative to each other, i.e. no mobility is allowed between these two parts.

## Claims

1. An electroporation device for injecting a product into an eye, said device comprising:
- a first part (10) comprising a rim (12) having the shape of a circle or of an arc of a circle, said circle having an axis X and a radius of greater than 5 mm and of less than 8 mm, so as to substantially correspond to the radius of the limbus (L) of the eye (O),
- a second part (30) mobile relative to the first part and guided by said first part, said second part comprising an invasive first electrode (40) comprising at least one needle having a length greater than 2 mm, the first electrode being designed to be connected electrically to a first terminal of an electrical generator, **characterised in that** the second part is guided in translation, relative to the first part, along a direction (T) corresponding to the direction of the invasive needle of the first electrode,
wherein the angle δ between a needle of the first electrode and a plane perpendicular to the axis X is less than 15°.

2. An electroporation device according to the preceding claim, wherein the first part has a spherical first part contact surface (60) born by a virtual sphere, the radius of curvature of said spherical contact surface ranging between 11 mm and 14 mm, so as to substantially correspond to the radius of curvature of the outside surface of said eye,
wherein the area of said spherical first part contact surface is preferably greater than 30 mm².

3. An electroporation device according to any one of the preceding claims,
wherein, the first invasive electrode comprises a set of parallel identical and rectilinear needles, extending along a common general direction (T), and electriclally connected to each other.

4. An electroporation device according to any one of the preceding claims,
wherein, the second part comprises a second electrode (50) having an electrically conductive spherical electrode contact surface (52) born by a virtual sphere, the radius of curvature of said electrode contact surface ranging between 11 mm and 14 mm, so as to substantially correspond to the radius of curvature of the outside surface of said eye, said second electrode being designed to be connected electrically to a second terminal of said electrical generator.

5. An electroporation device according to the preceding claim, wherein the rims of the first part and of the second electrode are coplanar at least in a position where the first part and the second part bear on a same virtual sphere, the radius of curvature of said virtual sphere ranging between 11 mm and 14 mm.

6. An electroporation device according to any one of the preceding claims, comprising an injection needle (40) which is not an electrode.

7. An electroporation device according to the immediately preceding claim, comprising means to guide said injection needle into a position adapted for the injection of the product.

8. An electroporation device according to any one of the preceding claims, comprising less than 5 injection needles.

9. An electroporation device according to any one of the preceding claims, comprising
- elastic means, for instance a spring, configured to force the movement of second part toward the first part, and/or the movement of the first electrode relative to the second electrode, and/or
- a mechanism to automatically change the position of the second part relative to the first part.

10. An electroporation device according to any one of the preceding claims, wherein a needle of the first electrode substantially extends in a plane including said axis X

11. An electroporation device according to any one of the claims 4 to 10, wherein the second electrode comprises a rim (62) having the shape of an arc of a circle, said circle having an axis X' and a radius (R') of greater than 5 mm and of less than 8 mm,
wherein the first electrode is guided between a remote position and a close position or is fixed relative to the second electrode,
wherein a needle of the first electrode extends permanently or in the close position at a distance *d* less than 10 mm from the rim (62) of said second electrode and/or at a distance d' less than 5 mm from said second electrode.

12. An electroporation device according to any one of the claims 4 to 11, wherein the second electrode comprises a rim (62) having the shape of an arc of a circle, said circle having an axis X' and a radius of greater than 5 mm and of less than 8 mm,
wherein the second electrode has a spherical contact surface, the radius of curvature of said spherical contact surface ranging between 11 mm and 14 mm,
wherein the first electrode is guided between a remote position and a close position or is fixed relative to the second electrode, a needle of the first electrode extending, or being guidable so as to extend, so as to protrude from the virtual sphere corresponding to the contact surface of the second electrode, and
wherein, permanently or in the close position, the distance *d"* between the rim (62) of said second electrode and the point (P) of said virtual sphere from which said needle protrudes or may protrude is less than 25 mm.

13. An electroporation device according to any one of the preceding claims, wherein the first electrode is guided between a remote position and a close position or is fixed relative to the second electrode,
wherein the second part comprises a second electrode which has a spherical electrode contact surface, the radius of curvature of said spherical contact surface ranging between 11 mm and 14 mm,
wherein a needle of the first electrode is electrically insulated over a part of its length, preferably by means of an insulating cover, so that, permanently or in the close position, no non-insulated part of said needle extends at a distance less than 1.0 mm from said spherical electrode contact surface.

## Patentansprüche

1. Elektroporationsvorrichtung zur Injektion eines Produkts in ein Auge, wobei die Vorrichtung das Folgende umfasst:
- einen ersten Teil (10), der einen Rand (12) in Form eines Kreises oder eines Kreisbogens aufweist, wobei der Kreis eine Achse X und einen Radius von mehr als 5 mm und von weniger als 8 mm aufweist, damit er im Wesentlichen dem Radius des Limbus (L) des Auges (O) entspricht,
- einen zweiten Teil (30), der bezüglich des ersten Teils beweglich ist und vom ersten Teil geführt wird, wobei der zweite Teil eine invasive erste Elektrode (40) umfasst, die mindestens eine Nadel mit einer Länge von mehr als 2 mm umfasst, wobei die erste Elektrode zur elektrischen Verbindung mit einer ersten Anschlussklemme eines elektrischen Generators ausgelegt ist,
**dadurch gekennzeichnet, dass** der zweite Teil in Translation bezüglich des ersten Teils entlang einer Richtung (T) geführt wird, die der Richtung der invasiven Nadel der ersten Elektrode entspricht,
wobei der Winkel δ zwischen einer Nadel der ersten Elektrode und einer Ebene senkrecht zur Achse X weniger als 15° beträgt.

2. Elektroporationsvorrichtung nach dem vorhergehenden Anspruch, wobei der erste Teil eine kugelförmige Kontaktfläche (60) des ersten Teils aufweist, die von einer virtuellen Sphäre getragen wird, wobei der Krümmungsradius der kugelförmigen Kontaktfläche im Bereich zwischen 11 mm und 14 mm liegt, damit er im Wesentlichen dem Krümmungsradius der Außenfläche des Auges entspricht,
wobei die Fläche der kugelförmigen Kontaktfläche des ersten Teils vorzugsweise größer als 30 mm² ist.

3. Elektroporationsvorrichtung nach einem der vorhergehenden Ansprüche, wobei die erste invasive Elektrode einen Satz von parallelen identischen und geradlinigen Nadeln umfasst, die sich entlang einer gemeinsamen allgemeinen Richtung (T) erstrecken und elektrisch miteinander verbunden sind.

4. Elektroporationsvorrichtung nach einem der vorhergehenden Ansprüche, wobei der zweite Teil eine zweite Elektrode (50) mit einer elektrisch leitfähigen kugelförmigen Elektrodenkontaktfläche (52) umfasst, die von einer virtuellen Sphäre getragen wird, wobei der Krümmungsradius der Elektrodenkontaktfläche im Bereich zwischen 11 mm und 14 mm liegt, damit er im Wesentlichen dem Krümmungsradius der Außenfläche des Auges entspricht, wobei die zweite Elektrode zur elektrischen Verbindung mit einer zweiten Anschlussklemme des elektrischen Generators ausgelegt ist.

5. Elektroporationsvorrichtung nach dem vorhergehenden Anspruch, wobei die Ränder des ersten Teils und der zweiten Elektrode zumindest in einer Position, in welcher der erste Teil und der zweite Teil auf einer selben virtuellen Sphäre liegen, koplanar sind, wobei der Krümmungsradius der virtuellen Sphäre im Bereich zwischen 11 mm und 14 mm liegt.

6. Elektroporationsvorrichtung nach einem der vorhergehenden Ansprüche, umfassend eine Injektionsnadel (40), die keine Elektrode ist.

7. Elektroporationsvorrichtung nach dem unmittelbar vorhergehenden Anspruch, umfassend Mittel zum Führen der Injektionsnadel in eine Position, die zur Injektion des Präparats ausgelegt ist.

8. Elektroporationsvorrichtung nach einem der vorhergehenden Ansprüche, umfassend weniger als 5 Injektionsnadeln.

9. Elektroporationsvorrichtung nach einem der vorhergehenden Ansprüche, umfassend
- elastische Mittel, beispielsweise eine Feder, die dazu ausgelegt sind, die Bewegung des zweiten Teils zum ersten Teil und/oder die Bewegung der ersten Elektrode bezüglich der zweiten Elektrode zu erzwingen, und/oder
- einen Mechanismus zur automatischen Veränderung der Position des zweiten Teils bezüglich des ersten Teils.

10. Elektroporationsvorrichtung nach einem der vorhergehenden Ansprüche, wobei sich eine Nadel der ersten Elektrode im Wesentlichen in einer Ebene erstreckt, welche die Achse X umfasst.

11. Elektroporationsvorrichtung nach einem der Ansprüche 4 bis 10, wobei die zweite Elektrode einen Rand (62) in Form eines Kreisbogens aufweist, wobei der Kreis eine Achse X' und einen Radius (R') von mehr als 5 mm und von weniger als 8 mm aufweist,
wobei die erste Elektrode zwischen einer entfernten Position und einer nahen Position geführt wird oder bezüglich der zweiten Elektrode fixiert ist,
wobei sich eine Nadel der ersten Elektrode permanent oder in der nahen Position in einem Abstand d von weniger als 10 mm vom Rand (62) der zweiten Elektrode und/oder in einem Abstand d' von weniger als 5 mm von der zweiten Elektrode erstreckt.

12. Elektroporationsvorrichtung nach einem der Ansprüche 4 bis 11, wobei die zweite Elektrode einen Rand (62) in Form eines Kreisbogens aufweist, wobei der Kreis eine Achse X' und einen Radius von mehr als 5 mm und von weniger als 8 mm aufweist,
wobei die zweite Elektrode eine kugelförmige Kontaktfläche aufweist, wobei der Krümmungsradius der kugelförmigen Kontaktfläche im Bereich zwischen 11 mm und 14 mm liegt,
wobei die erste Elektrode zwischen einer entfernten Position und einer nahen Position geführt wird oder bezüglich der zweiten Elektrode fixiert ist, wobei sich eine Nadel der ersten Elektrode so erstreckt, oder so geführt werden kann, dass sie sich so erstreckt, dass sie von der virtuellen Sphäre vorragt, entsprechend der Kontaktfläche der zweiten Elektrode, und
wobei permanent oder in der nahen Position der Abstand *d*'' zwischen dem Rand (62) der zweiten Elektrode und dem Punkt (P) der virtuellen Sphäre, von der die Nadel vorragt oder vorragen kann, weniger als 25 mm beträgt.

13. Elektroporationsvorrichtung nach einem der vorhergehenden Ansprüche, wobei die erste Elektrode zwischen einer entfernten Position und einer nahen Position geführt wird oder bezüglich der zweiten Elektrode fixiert ist,
wobei der zweite Teil eine zweite Elektrode mit einer kugelförmigen Elektrodenkontaktfläche umfasst, wobei der Krümmungsradius der kugelförmigen Kontaktfläche im Bereich zwischen 11 mm und 14 mm liegt,
wobei eine Nadel der ersten Elektrode über einen Teil ihrer Länge elektrisch isoliert ist, vorzugsweise durch eine Isolierabdeckung, so dass sich kein nicht isolierter Teil der Nadel permanent oder in der nahen Position über eine Strecke von weniger als 1,0 mm von der kugelförmigen Elektrodenkontaktfläche erstreckt.

## Revendications

1. Dispositif d'électroporation pour injecter un produit dans un oeil, ledit dispositif comprenant :
- une première pièce (10) comprenant un pourtour (12) ayant la forme d'un cercle ou d'un arc de cercle, ledit cercle ayant un axe X et un rayon supérieur à 5 mm et inférieur à 8 mm, de façon à correspondre sensiblement au rayon du limbe (L) de l'oeil (O),
- une seconde pièce (30) mobile par rapport à la première pièce et guidée par ladite première pièce, ladite seconde pièce comprenant une première électrode (40) invasive comprenant au moins une aiguille ayant une longueur supérieure à 2 mm, la première électrode étant conçue pour être raccordée électriquement à la première borne d'un générateur électrique, **caractérisé en ce que** la seconde pièce est guidée en translation, par rapport à la première pièce, suivant une direction (T) correspondant à la direction de l'aiguille invasive de la première électrode,
dans lequel l'angle δ entre une aiguille de la première électrode et un plan perpendiculaire à l'axe X est inférieur à 15°.

2. Dispositif d'électroporation selon la revendication précédente, dans lequel la première pièce comporte une surface de contact (60) de première pièce sphérique portée par une sphère virtuelle, le rayon de courbure de ladite surface de contact sphérique allant de 11 mm à 14 mm, de façon à correspondre sensiblement au rayon de courbure de la surface extérieure dudit oeil, dans lequel la superficie de ladite surface de contact de première pièce sphérique est de préférence supérieure à 30 mm².

3. Dispositif d'électroporation selon l'une quelconque des revendications précédentes, dans lequel la première électrode invasive comprend un jeu d'aiguilles parallèles identiques et rectilignes, s'étendant suivant une direction générale (T) commune et raccordées électriquement l'une à l'autre.

4. Dispositif d'électroporation selon l'une quelconque des revendications précédentes, dans lequel la seconde pièce comprend une seconde électrode (50) comportant une surface de contact (52) d'électrode sphérique électro-conductrice, portée par une sphère virtuelle, le rayon de courbure de ladite surface de contact d'électrode allant de 11 mm à 14 mm, de façon à correspondre sensiblement au rayon de courbure de la surface extérieure dudit oeil, ladite seconde électrode étant conçue pour être raccordée électriquement à la seconde borne dudit générateur électrique.

5. Dispositif d'électroporation selon la revendication précédente, dans lequel les pourtours de la première pièce et de la seconde électrode sont coplanaires au moins dans une position où la première pièce et la seconde pièce s'appuient sur une même sphère virtuelle, le rayon de courbure de ladite sphère virtuelle allant de 11 mm à 14 mm.

6. Dispositif d'électroporation selon l'une quelconque des revendications précédentes, comprenant une aiguille d'injection (40) qui n'est pas une électrode.

7. Dispositif d'électroporation selon la revendication immédiatement précédente, comprenant un moyen pour guider ladite aiguille d'injection dans une position adaptée à l'injection du produit.

8. Dispositif d'électroporation selon l'une quelconque des revendications précédentes, comprenant moins de 5 aiguilles d'injection.

9. Dispositif d'électroporation selon l'une quelconque des revendications précédentes, comprenant .
- un moyen élastique, par exemple un ressort, configuré pour forcer le mouvement de la seconde pièce vers la première pièce et/ou le mouvement de la première électrode par rapport à la seconde électrode, et/ou
- un mécanisme pour modifier automatiquement la position de la seconde pièce par rapport à la première pièce.

10. Dispositif d'électroporation selon l'une quelconque des revendications précédentes, dans lequel une aiguille de la première électrode s'étend sensiblement dans un plan comprenant ledit axe X.

11. Dispositif d'électroporation selon l'une quelconque des revendications 4 à 10, dans lequel la seconde électrode comprend un pourtour (62) ayant la forme d'un cercle ou d'un arc de cercle, ledit cercle ayant un axe X' et un rayon (R') supérieur à 5 mm et inférieur à 8 mm,
dans lequel la première électrode est guidée entre une position éloignée et une position proche ou est fixe par rapport à la seconde électrode,
dans lequel une aiguille de la première électrode s'étend de façon permanente ou en position proche à une distance d inférieure à 10 mm du pourtour (62) de ladite seconde électrode et/ou à une distance d' inférieure à 5 mm de ladite seconde électrode.

12. Dispositif d'électroporation selon l'une quelconque des revendications 4 à 11, dans lequel la seconde électrode comprend un pourtour (62) ayant la forme d'un cercle ou d'un arc de cercle, ledit cercle ayant un axe X' et un rayon supérieur à 5 mm et inférieur à 8 mm,
dans lequel la seconde électrode a une surface de contact sphérique, le rayon de courbure de ladite surface de contact sphérique allant de 11 mm à 14 mm,
dans lequel la première électrode est guidée entre une position éloignée et une position proche ou est fixe par rapport à la seconde électrode, une aiguille de la première électrode s'étendant ou pouvant être guidée de façon à s'étendre, afin de dépasser de la sphère virtuelle correspondant à la surface de contact de la seconde électrode, et
dans lequel, de façon permanente ou en position proche, la distance d" entre le pourtour (62) de ladite seconde électrode et le point (P) de ladite sphère virtuelle à partir duquel ladite aiguille dépasse ou peut dépasser est inférieure à 25 mm.

13. Dispositif d'électroporation selon l'une quelconque des revendications précédentes, dans lequel la première électrode est guidée entre une position éloignée et une position proche ou est fixe par rapport à la seconde électrode,
dans lequel la seconde pièce comprend une seconde électrode qui comporte une surface de contact d'électrode sphérique, le rayon de courbure de ladite surface de contact sphérique allant de 11 mm à 14 mm,
dans lequel une aiguille de la première électrode est isolée électriquement sur une partie de sa longueur, de préférence au moyen d'une enveloppe isolante, si bien que, de façon permanente ou en position proche, aucune partie non isolée de ladite aiguille ne s'étend à une distance inférieure à 1,0 mm de ladite surface de contact d'électrode sphérique.
